# EUROPEAN PATENT APPLICATION

(11) **EP 4 806 357 A1**
(43) Date of publication of application: **16.09.2026**
(21) Application number: 25162484.7
(22) Date of filing: 10.03.2025
(51) Int. Cl.: A61B 6/00, A61B 6/10

(54) **A RAIL SYSTEM FOR A CEILING MOUNTED IMAGING SYSTEM**

(71) Applicant: Koninklijke Philips N.V., 5656 AE Eindhoven (NL)
(72) Inventor: DRIES, Johan Juliana, Eindhoven (NL); VAN DER WACHT, Rogier Sean, Eindhoven (NL); GAST, Tomas, Eindhoven (NL); VAN HEESCH, Frank, Eindhoven (NL); HENDRIKS, Bernardus Hendrikus Wilhelmus, Eindhoven (NL)
(74) Representative: Philips Intellectual Property & Standards

(57) **Abstract**

A rail system for a ceiling mounted imaging component comprises a first pair of elongated rails 10 positioned in parallel and configured for slideably coupling an imaging component 60, 70, 80 thereto. The rail system further includes a second pair of elongated rails 20 positioned in parallel to the first pair and configured for slideably coupling a monitor ceiling suspension 65 thereto. The rails of the second pair are spatially separated from each other by the first pair of rails and a curved rail part 30 couples the rails of the second pair to form a loop partly or wholly the imaging component and enabling a monitor 66 attached to the monitor ceiling suspension to be moved over the loop around the patient bed 67 to a position that is not conflicting with a movement 61 of the imaging component and provides a good viewing for a physician 115. The rail system further includes a support structure comprising rods 50 and rod interface blocks for coupling the first pair of rails, the second pair of rails and the curved rail part to a ceiling 55.

## Description

### FIELD OF THE INVENTION

The present invention relates to a rail system for a ceiling mounted medical imaging component, to a medical imaging system comprising the rail system for a ceiling mounted imaging component and to a method of assembling a rail system for a medical imaging component.

### BACKGROUND OF THE INVENTION

A catheterization laboratory, also referred to as cathlab, is an examination room in a hospital equipped with X-ray imaging equipment and one or more monitors to follow with X-ray images the progress of an interventional procedure. In the interventional procedure, by insertion and manipulation of suitable catheters or guidewires at the appropriate anatomical location, a patient is treated with minimal invasion of the body. The catheter or guidewire is slowly and gradually moved up towards a target anatomy by an interventionalist. The location of the catheter or guidewire is tracked by fluoroscopy and shown on a monitor. To provide different viewing directions of (for example) a lesion in a vessel of a patient, X-ray images may be taken from different angles. To enable this X-ray imaging systems have been developed with a moveable X-ray tube and X-ray detector, wherein the X-ray tube and the X-ray detector are aligned in a C-arc. A ceiling-mounted x-ray system typically involves rails or tracks mounted on the ceiling that allow the X-ray tube and detector to be moved horizontally. Next to the C-arc also the monitor needs to be moveably coupled to the ceiling to allow a positioning of the monitor out of the path of a moving C-arc or to a position that allows the interventionalist to have a good view on the X-ray images displayed on the monitor. The requirement to have also a moveable monitor further adds to complexity and cost.

### SUMMARY OF THE INVENTION

It is an object to provide a rail system for a ceiling mounted medical imaging component which is less complex.

The object is achieved by the subject matter of the independent claims; further embodiments are incorporated in the dependent claims.

According to the present disclosure a rail system for a ceiling mounted medical imaging component is provided. The rail system comprises a first pair of elongated rails positioned in parallel. The first pair of elongated rails is arranged for slideably coupling the medical imaging component. The rail system further comprises a second pair of elongated rails positioned in parallel to the first pair. The second pair of elongated rails is arranged for slideably coupling a monitor ceiling suspension. The rails of the second pair are spatially separated from each other by the first pair of rails. The rail system further comprises a curved rail part that couples the rails of the second pair of elongated rails such that the curved rail part together with the rails of the second pair rails form a loop which partly or wholly encloses the first pair of rails. The curved rail part is arranged to allow a sliding movement of the monitor ceiling suspension over the loop. The loop includes the rails of the second pair which are spatially separated from each other and coupled at one end with the curved rail part to result in a substantially U-shaped track around the first pair of rails, or a part of the first pair of rails. The rail system further comprises a support structure that may be coupled to the ceiling. The first pair of rails, second pair of rails and curved rail part are coupled to the support structure.

The rail system as claimed enables a flexible movement of a monitor ceiling suspension over the loop around the medical imaging component which may be moved to a suitable location over the first pair of rails. The curved rail part, the first and second pair of rails are coupled to the support structure which is coupled to the ceiling of a hospital room. The rail system integrates the rails needed for the movement of the monitor ceiling suspension as well as the rails needed for movement of the imaging component which results in a rail system that is less complex.

In an embodiment the rail system comprises a further curved rail part coupled to the U-shaped track created by the second pair of elongated rails and the curved rail part. One end of each of the rails of the second pair is coupled with the curved rail part and the other end of each of the rails is coupled with the further curved rail part. Together the curved rail part, the second pair of elongated rails and the further curved rail part form a closed loop around the first pair of elongated rails, thereby enabling a movement of the monitor ceiling suspension around the imaging component regardless of where it may have been positioned on the first pair of rails. A medical imaging system comprising this rail system has the advantage that a monitor coupled to the monitor ceiling suspension may be optimally positioned enhancing the ergonomics. In contrast with known monitor ceiling suspensions used in cathlabs the rail system of the present disclosure enables the monitor ceiling suspension, and the monitor coupled thereto to be positioned on any side of a patient and on any side of the ceiling mounted imaging component.

In an embodiment of the rail system the support structure comprises a first plurality of rod interface blocks and a second plurality of rods. Each rod interface block is coupled to the first pair of elongated rails, or to the second pair of elongated rails or to the curved rail part or to the further curved rail part. A rod interface block may for example be coupled to a rail of the first pair of elongated rails and a neighboring rail of the second pair of elongated rails. Each rod is arranged for coupling a rod interface block to the ceiling.

A further aspect of the present disclosure provides a medical imaging system comprising the rail system as discussed above. The medical imaging system further includes a carriage that is slideably coupled to the first pair of elongated rails and the imaging component of the medical imaging system is coupled to the carriage. The carriage allows a horizontal translational movement of the medical imaging component. The medical imaging system further comprises a monitor ceiling suspension that is slideably coupled to the second pair of elongated rails and a monitor that is coupled to the monitor ceiling suspension. The monitor position can be changed by moving the monitor ceiling suspension over the second pair of elongated rails and the curved part (and when applicable the further curved part) which are looped around the first pair of rails that is carrying the carriage and the imaging component coupled to the carriage. The medical imaging system further comprises a patient bed to support a patient during a clinical procedure. Such a medical imaging system is typically positioned in an examination room of a hospital.

An imaging component as defined in this disclosure may include both the X-ray source and X-ray detector or only an X-ray source. A first example of a ceiling mounted imaging component is a C-arc including an X-ray source and an X-ray detector. A second example of a ceiling mounted imaging component is an upper detector robot holding an X-ray detector. In contrast with the first example of the C-arc the X-ray source is floor mounted with a separate mechanical arrangement as will be discussed in more detail below.

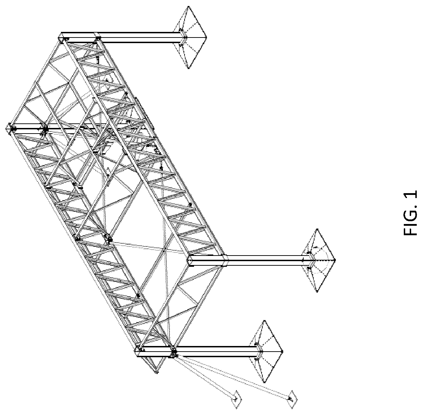

In an embodiment of the rail system the imaging component comprises a C-arc including an X-ray source and an X-ray detector. The ceiling of the examination room must be fit to carry the weight of the mechanical construction needed for a moveable C-arc (e.g. the rail system) and the C-arc itself. Whether the ceiling is fit to carry the weight depends on the building in which the cathlab is to be installed and sometimes a separate ceiling construction as shown in Fig. 1 is needed which has the required mechanical characteristics for coupling the ceiling mounted C-arc thereto. This adds to complexity and cost.

X-ray systems are provided that have individual supports for source and detector. For example, WO 2020/089272 A1 describes a system with two robot arms holding the X-ray source and the X-ray detector.

In an embodiment of the medical imaging system the imaging component comprises an upper detector robot for movably holding an X-ray detector, the upper detector robot being coupled to the carriage. An advantage is that a detector robot holding an X-ray detector has less weight than a C-arc including an X-ray tube and an X-ray detector. A lower weight of the imaging component also reduces the mechanical requirements that the rail system for a ceiling mounted imaging system must comply with, which may provide cost reduction opportunities. The medical imaging system further comprises a patient bed to be positioned on a floor of the cathlab and a lower source robot for movably holding an X-ray source. The lower source robot also is positioned on the floor.

In a further embodiment the medical imaging system further comprises a carriage drive arrangement which is arranged to control a translational horizontal movement of the carriage over the first pair of elongated rails. This enables the movement of the imaging component to a position needed for obtaining an X-ray of a part of the body.

In a further embodiment the medical imaging system further comprises a monitor drive arrangement arranged to control a movement of the monitor ceiling suspension over the loop around the first pair of elongated rails. The monitor drive arrangement may be further arranged to control the monitor ceiling suspension to move the monitor in a direction closer to the patient bed or further away from the patient bed. This allows the interventionalist to move the monitor to a position that gives an optimal view on the monitor.

In a further embodiment the medical imaging system further comprises a control system configured to control any one or more of the carriage drive arrangement, the monitor drive arrangement, the upper detector robot, the lower source robot. The medical imaging system may for example comprise a touch screen user interface for receiving input on a user selected position of the radiation detector, the radiation source or the monitor. Or the interventionalist may use the touchscreen to provide input on a user selected clinical procedure having a relating "default" position for the radiation detector, the radiation source or the monitor. The control system is configured to control the position of the radiation detector, the radiation source or the monitor in dependence of the received input.

In a further embodiment the medical imaging system includes position sensors for determining the position of the carriage and the monitor ceiling suspension. The carriage drive arrangement includes a carriage position sensor arranged to determine the position of the carriage on the first pair of rails and the monitor drive arrangement includes a monitor position sensor arranged to determine the position of the monitor ceiling suspension on the loop. The control system is configured to process data received from the carriage position sensor and the monitor position sensor and control any one or more of the carriage drive arrangement, the monitor drive arrangement, the upper detector robot, the lower source robot in dependence of the processed data.

In a further embodiment the medical system further comprises a camera. The control system is configured to process the image data obtained by the camera and control any one or more of the carriage drive arrangement, the monitor drive arrangement, the detector robot or the source robot in dependence of the processed image data. The control system is configured to process the images obtained with the camera and determine a potential position conflict between a desired and present position of anyone or more of the X-ray detector, upper detector robot, X-ray source, lower source robot, monitor, monitor ceiling suspension, patient bed, patient and interventionalist. The control system is further configured to change the position of the monitor ceiling suspension on the loop (created by the rails of the second pair and the curved rail part and, when applicable, the further curved rail part), the carriage, the upper detector robot or the lower source robot in dependence of the determined potential position conflict and prevent a collision.

A further aspect of the present disclosure provides a method of assembling a rail system for a ceiling mounted medical imaging component. The method comprises:
coupling a first pair of elongated rails to a support structure wherein the rails of the first pair of elongated rails are positioned in parallel and configured for slideably coupling the medical imaging component thereto;
coupling a second pair of elongated rails to the support structure wherein the rails of the second pair are positioned in parallel to the first pair and configured for slideably coupling a monitor ceiling suspension, and wherein the rails of the second pair are spatially separated from each other by the first pair of rails;
coupling a curved rail part to the rails of the second pair of elongated rails, wherein the curved rail part together with the second pair or elongated rails form a loop that partly or wholly encloses the first pair of rails and wherein the curved rail part is arranged to allow a sliding movement of the monitor ceiling suspension over the full length of the loop;
coupling the curved rail part to the support structure;
coupling the support structure to a ceiling;
slideably coupling an imaging component to the first pair of rails;
slideably coupling the monitor ceiling suspension to the second pair of rails.

The resulting rail system integrates the rails for the moveable monitor ceiling suspension and the rails for the moveable imaging component. The integrated rail system enables cost reduction and includes desired functionality of moving the imaging component to a desired position to perform a medical imaging procedure, and moving a monitor coupled to a monitor ceiling suspension to a user preferred position.

In an embodiment the method of assembling a rail system for a ceiling mounted medical imaging component further comprises
coupling a further curved rail part to the rails of the second pair of elongated rails, wherein the further curved rail part, the curved rail part and the second pair of elongated rails coupled together form a closed loop around the first pair of rails,
coupling the further curved rail part to the support structure.

The rail system that is obtained with this method of assembling allows the monitor ceiling suspension and any monitor coupled thereto to be positioned on either side of a patient bed and on either side of the imaging component which provides enhanced flexibility and ergonomics.

In a further embodiment the method of assembling a rail system for a ceiling mounted medical imaging component further comprises a coupling of each rail of the second pair of elongated rails with its closest neighboring rail of the first pair of elongated rails. This method provides a compact rail system with additional stiffness and strength.

In a further embodiment the method the coupling of a rail of the first pair or the second pair to the support structure comprises
coupling the rail to at least one rod interface block, and
coupling each of the at least one rod interface block with at least one rod to the ceiling.

These and other aspects of the present disclosure will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a portal construction;
Fig. 2 shows an example of a part of an integrated rail system;
Fig. 3 shows a further example of a part of an integrated rail system in which the rails for the moveable monitor ceiling suspension are forming a loop around the rails for the moveable medical imaging component;
Fig. 4 shows a further example of a part of an integrated rail system in which each elongated rail used for the monitor ceiling suspension is coupled to its neighboring rail used for the carriage carrying the imaging component;
Fig. 5 shows an example of a part of an integrated rail system having curved rail part including a metal plate for additional stability;
Fig. 6 shows an example of an integrated rail system shown earlier in Fig. 2 including rod interface blocks (the rods which are included in the support structure are not shown);
Fig. 7 shows another example of an integrated rail system shown earlier in Fig. 4 including rod interface blocks (the rods which are included in the support structure are not shown);
Fig. 8 shows an example of a cross section of an integrated rail system that is coupled to a ceiling;
Fig. 9 shows another view on the integrated rail system earlier shown in Fig. 8 (the curved rail part connecting the rails of the second pair is not shown);
Fig. 10 shows an example of a medical imaging system including an upper detector robot slideably coupled to an integrated rail system (the rod interface blocks are not shown);
Fig. 11 shows an example of a medical imaging system including an upper detector robot and a lower source robot, wherein the upper detector and a monitor ceiling suspension are slideably coupled to an integrated rail system;
Fig. 12 shows the basic steps of an example of a method of assembling a rail system for a medical imaging system.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of", when preceding a list of elements, modify the entire list of elements and do not modify the individual elements of the list.

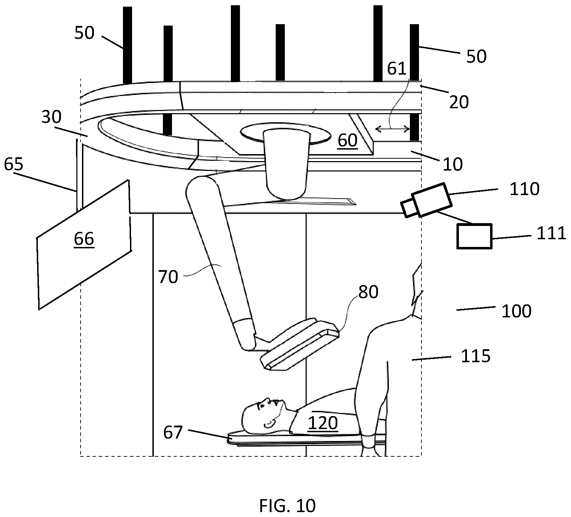

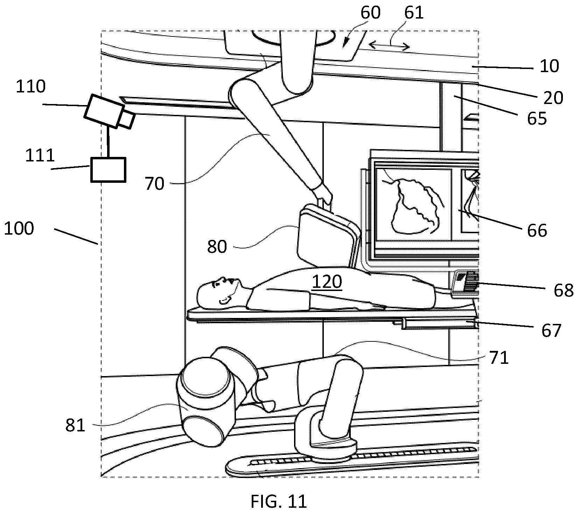

The present disclosure relates to a rail system for a ceiling mounted medical imaging component. A medical imaging component as defined in this disclosure may include both the X-ray source and X-ray detector or only an X-ray source or only an X-ray detector. For example, a ceiling mounted imaging component may be a ceiling mounted C-arc including the X-ray source and the X-ray detector. An example of a ceiling mounted imaging system is the Philips Azurion with FlexArm which includes a ceiling mounted imaging system. Another example of a ceiling mounted imaging component is an upper detector robot 70 holding an X-ray detector 80 as shown in the example of Fig. 10 or 11. In contrast with the earlier example of the C-arc the X-ray source 81 is floor mounted with a lower source robot 71 holding the X-ray source as will be discussed in more detail below. An example of a medical imaging system including a ceiling mounted upper detector robot is disclosed in WO 2020/089272 A1 describing an Xray imaging arrangement with two separate robot arms holding the X-ray source and the X-ray detector.

The term "lower source robot" as used in the present disclosure relates to a robot for moving and holding the X-ray source. The robot is mounted to the floor and the X-ray source is mounted at the free end of the robot. The robot may have the form of an arm with several movable joints that allow a high degree of freedom of movement.

The term "upper detector robot" as used in the present disclosure relates to a robot for moving and holding the X-ray detector. The robot is mounted to the ceiling and the X-ray detector is mounted at the free end of the robot. The robot may have the form of an arm with several movable joints that allow a high degree of freedom of movement.

A rail system for a ceiling mounted imaging component according to the present disclosure comprises: a first pair of elongated rails 10 positioned in parallel and configured for slideably coupling the imaging component 70, 80 thereto; a second pair of elongated rails 20 positioned in parallel to the first pair and configured for slideably coupling a monitor ceiling suspension 65 thereto, the rails of the second pair being spatially separated from each other by the first pair of rails; a curved rail part 30 coupling the rails of the second pair of elongated rails, the curved rail part together with the second pair or elongated rails forming a loop partly or wholly enclosing the first pair of rails; the curved rail part being arranged to allow a sliding movement of the monitor ceiling suspension over the full length of the loop; a support structure coupleable to the ceiling 55 of an examination room, the first pair of rails, second pair of rails and curved rail part being coupled to the support structure.

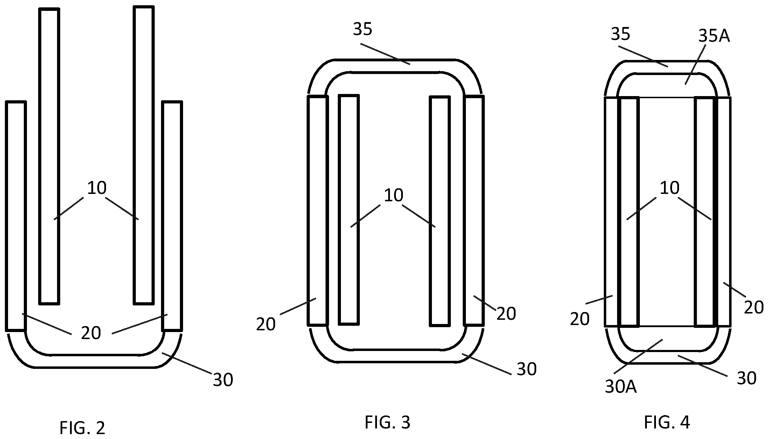

The term "loop" as used in the present disclosure includes the rails of the second pair 20 which are spatially separated from each other and coupled at one end with a curved rail part 30 to result in a substantially U-shaped track around the first pair of rails 10, or a part of the first pair of rails as shown in see Fig. 2. The loop may be closed by coupling a further curved rail part 35 at the "free" ends of the rails of the second pair. The closed loop fully encloses the first pair of rails as schematically shown in Fig. 3 and Fig. 4.

Existing rail systems may need a separate mechanical construction for the monitor ceiling suspension which involves additional room preparation and results in added cost. The rail system of the present disclosure integrates the rails for the monitor ceiling suspension in the mechanical construction for the ceiling mounted imaging system thereby reducing the required room preparation and cost.

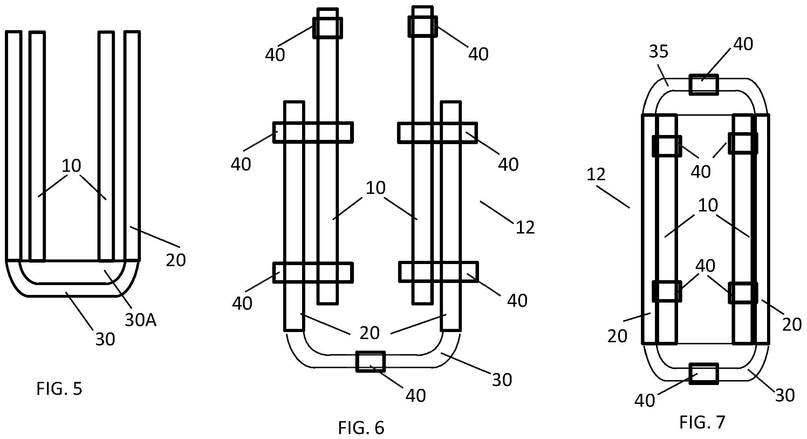

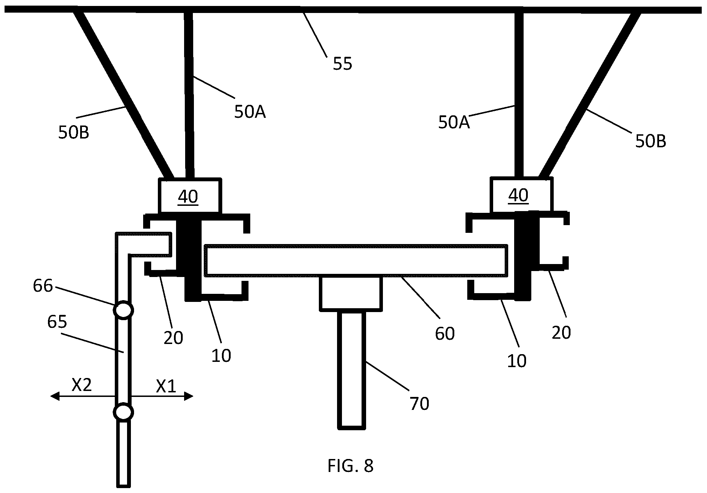

Figs. 2 - 7 shows a schematic example of a part of such an integrated rail system for a ceiling mounted imaging system. The rail system includes a first pair of rails 10 that is configured for slideably coupling an imaging component thereto. Fig. 10 shows an example of such a slideably coupled imaging component comprising an upper detector robot 70 coupled to a carriage 60 arranged to slide over the first pair of rails 10. Returning to Fig. 2, the rail system further includes a second pair of elongated rails 20 positioned in parallel to the first pair 10. The rails of the second pair may be longer or shorter than the rails of the first pair. The rails of the second pair are spatially separated from each other by the first pair of rails. An end part of each rail of the second pair is coupled to a curved rail part 30 such that the rails of the second pair are coupled to each other by the curved rail part creating a loop that encloses part of the first pair of rails, as shown in Fig. 2 or Fig. 6. The rails of the second pair 20 and the coupled curved rail part are configured for slideably coupling a monitor ceiling suspension 65 (not shown in Fig. 2). The loop created by the rails of the second pair coupled at one end with the curved rail part result in a substantially U-shaped track around the first pair of rails, or a part of the first pair of rails as shown in Fig. 2 and 6. The monitor ceiling suspension is slideable over the track. Fig. 8 schematically shows a cross section of the rail system including the monitor ceiling suspension 65 slideably coupled to a rail of the second pair of rails 20. The rails of the first pair 10 are spatially separated from each other to accommodate the slideable coupling of a carriage 60 which carries the upper detector robot 70, only part of which is shown in Fig. 8.

The rail system may further comprise a further curved rail part 35 coupling the rails of the second pair of elongated rails, wherein the further curved rail part 35, the curved rail part 30 and the second pair of elongated rails 20 coupled together create a closed loop around the first pair of rails 10 as schematically shown in Fig. 3, 4 and 7.

Fig. 3 shows a rail system in which both ends of each rail of the second pair are coupled with curved rail parts 30, 35. One end of each rail of the second pair of rails 20 is coupled with a curved rail part 30. The other end of each rail of the second pair is coupled with a further curved rail part 35. The rails of the second pair 20 together with the curved and further curved rail parts 30, 35 create a closed loop around the first pair of rails 10 and make it possible that the monitor ceiling suspension (not shown in Fig. 3) that is slideably coupled to the second pair of rails is moveable around the imaging component which is horizontally slideable over the first pair of rails 10 to any position within the closed loop. As an example, the rails of the first and second pair of rails may be created using extrusion profiles or milled profiles using aluminum, steel or other materials.

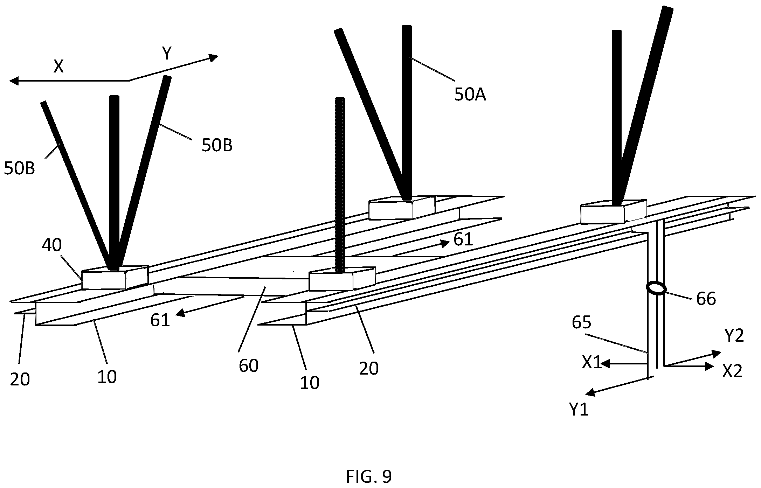

In Fig. 4 an example of a rail system is shown in which each rail of the second pair 20 is coupled "side to side" with a closest neighboring rail of the first pair 10. A cross section of this arrangement is also shown in Fig. 8 or schematically in 3-D in Fig. 9. This arrangement may provide mechanical advantages (e.g. enhanced stiffness) to the rail system.

Fig. 5 shows an example of a curved rail part 30 coupled to the ends of the rails of the second pair of rails 20 wherein the curved rail part includes a plate 30A which is coupled to the curved rail. The plate 30A is also mechanically coupled to an end of each rail of the first pair of rails 10 to provide enhanced stiffness to the rail system. Fig. 4 and Fig. 7 show examples of a rail system in which both the curved rail part 30 as well as the further curved rail part 35 includes a plate 30A, 35A.

The support structure included in the rail system comprises a first plurality of rod interface blocks 40. Each rod interface block is coupled to a rail of the first pair of elongated rails 10, or to a rail of the second pair of elongated rails 20 or to the curved rail part 30 or to the further curved rail part 35. The rail system further includes a second plurality of rods 50 arranged for coupling each of the rod interface blocks to the concrete ceiling 55 of an examination room.

The support structure couples each of the rails to the ceiling. The first plurality of rod interface blocks 40 may provide regular spaced suspension points for the first and second pair of rails 10, 20. Examples of the use of these rod interface blocks 40 are shown in Fig. 6 to 9. A rod interface block 40 may for example be coupled to one rail of a pair of rails 10, 20, or to two neighboring rails (a rail of the second pair 20 that is closest to a rail of the first pair of rails 10), or to two rails of the first pair (not shown), or to a curved rail part 30, or to a further curved rail part 35 as shown in the integrated rail system 12 of Figs. 6 and 7. The location and number of rod interface blocks depend on the mechanical characteristics of the used materials and the mechanical requirements for the rail system 12 such as levelness, strength and stiffness for the relevant degrees of freedom (e.g. the X and Y direction as shown in Fig. 9). Each rod interface block 40 may be coupled with at least one rod 50A, 50B to the ceiling 55 as shown in Fig. 8. A rod interface block may for example be realized by a milled steel block.

In the embodiment schematically shown in Fig. 8 the support structure includes at least two rods 50A, 50B that are positioned under an angle, for example of about 45 degrees with respect to a vertical line from ceiling to floor, to provide enhanced stiffness to the rail system in the horizontal X-Y plane (see Fig. 9). An interface block 40 may be coupled with two or more rods 50, 50A, 50B to the ceiling 55, the two or more rods including a first rod 50A being positioned in a vertical direction and a second rod 50B positioned under an angle of about 45 degrees with respect to the first rod. Fig. 9 schematically shows a further example of a support structure including non-vertically positioned rods 50B in the X- and Y- directions.

In an embodiment of the support structure the rods have an adjustable length to enable the positioning of the rail system in examination rooms with different ceiling heights or even steps in the ceiling height within one room.

In an embodiment of the support structure, the angle of the rods 50B with the vertical direction is larger and might even be 90 degrees such that these rods are horizontal and connecting to the walls of the room, rather than the ceiling while still serving the purpose of providing stiffness in the horizontal X- and Y-directions.

In a further embodiment the support structure includes steel profiles to provide the required strength and stiffness to the rail system. An example of these profiles are Unistrut profiles such as known from https://www.unistrut.co.uk/products/support-systems/single-and-double-channel.

Fig. 9 shows a 3D schematical drawing of part of the rail system (shown without the curved rail parts for explanation purpose only). Each rail of the second pair 20 is side to side coupled with its closest neighboring rail of the first pair 10. The "side to side" coupled rails are coupled to multiple rod interface blocks 40 (Fig. 9 only shows a limited number for explanation purpose only) and each rod interface block 40 is coupled with at least one rod 50A, 50B to a ceiling (not shown in Fig. 9). A carriage 60 is slideable in the rails of the first pair 10 of rails. A monitor ceiling suspension 65 is slideable in a rail of the second pair of rails. The monitor ceiling suspension 65 may further comprise one or more moveable joints 66 to enable the movement of a coupled monitor (not shown) in a direction X1, X2 perpendicular to the sliding direction Y1, Y2. In Fig. 11 can be seen that this makes it possible for the interventionalist to move the monitor 66 closer or further away from the patient table 67 (see also X1, X2 direction in Fig. 8, Fig. 9 which is a direction towards or away from a patient table (not shown) which is positioned under the rail system). The monitor ceiling suspension 65 is further arranged to rotate the monitor 66 dependent on the position of the interventionalist to have an optimal viewing angle. The rotation of the monitor as well as the movement of the monitor towards or away from the patient bed may be motorized and under control of a control system 111 shown in Fig. 10 and further discussed below.

The rail system has a large opening between the rails of the first pair of elongated rails 10. The opening is advantageously positioned above the patient bed as is most clearly seen in Fig. 10 and facilitates a laminar downflow air system which often is a requirement in a hospital setting in which hybrid procedures or surgical procedures (under image guidance) are executed.

A further advantage of the rail system is that the included parts, e.g. the elongated rails, the curved rail parts, the rod interface blocks and the rods may be created using well-known manufacturing techniques thereby providing cost advantages.

According to a further aspect of this disclosure a medical imaging system 100 comprising the rail system as discussed above is provided. An example of such a medical system is shown in Fig. 10. The medical system comprises a carriage 60 slideably coupled to the first pair of elongated rails 10, an imaging component 70, 80 of the medical imaging system coupled to the carriage, a monitor ceiling suspension 65 slideably coupled to the second pair of elongated rails 20, and a monitor 66 coupled to the monitor ceiling suspension 65. An advantage of the rail system included in the medical imaging system is that the positioning of the monitor ceiling suspension 65 is not limited by the position of the carriage 60 which provides that the monitor 66 can be positioned at both sides of the patient table or near the head side of the patient table 67 as shown in Fig. 10.

In an embodiment of the medical imaging system the imaging component comprises a C-arc including an X-ray detector and an X-ray source. Such a medical system may for example be used in a cathlab.

The C-arc including both the imaging source and the imaging detector needs to be moveably coupled to the ceiling of an examination room. A rail system including the C-arc coupled to it may have a total weight exceeding 1200kg. Fig. 1 schematically shows an example of a known portal construction which may be used when the ceiling in a hospital room is not strong enough to carry the total weight of the rail system including the coupled C-arc. The triangular shaped beam arrangements in the portal construction are used to create stiffness and strength. The portal construction is mounted on the floor. Alternatively, arrangements are possible in which a portal is bolted against the walls of the examination room. These mechanical constructions add to complexity, installation time and cost.

In a further embodiment the medical imaging system comprises an upper detector robot 70 for movably holding an X-ray detector 80 wherein the upper detector robot is coupled to the carriage 60. The medical imaging system further comprises a patient bed 67 to be positioned on a floor and a lower source robot 71 for movably holding an X-ray source 81 wherein the source robot to be positioned on the floor.

The above discussed embodiment of the imaging system including an upper detector robot 70 instead of a C-arc has the advantage of a reduced weight of the imaging component that is mounted to the ceiling. An example of such a medical imaging system is shown in Fig. 11 having individual supports for X-ray source 81 and X-ray detector 80 with a lower source robot 71 and an upper detector robot 70. The lower source robot 71 and the upper detector robot 70 are used to separately position the source and the detector.

Fig. 10 shows an example of the embodiment of the medical imaging system including an upper detector robot wherein the medical imaging system comprises the disclosed rail system as previously discussed. The curved rail part 30 couples the rails of second pair of rails 20 creating a loop around the rails of the first pair of rails 10. Each rail of the first pair of rails is coupled side to side with its closest neighboring rail of the second pair of rails, similar as discussed earlier for the rail system shown in Fig. 4 and 7. A carriage 60 is slideably coupled to the first pair of rails enabling a horizontal translation 61 of the carriage. An upper detector robot 70 holding an X-ray detector 80 is coupled to the carriage. The first and second pair of rails are coupled to rod interface blocks (not visible) and the rod interface blocks are coupled with rods 50 to a ceiling (not shown). The detector 80 coupled to the upper detector robot is positioned in the vicinity of a patient positioned on a patient bed 67.

In a further embodiment the medical imaging system may include one or more cameras 110 to capture images of the medical imaging system and interventionalist 115 in the environment around it and a control system 111 processing the images obtained with the camera. The control system comprises one or more processors running image recognition software configured to identify one or more persons 115 and one or more objects such as the imaging component 70, 80 or patient bed 67 in the received images and determine respective locations of the one or more persons 120, 115 and the one or more objects in the environment of the medical imaging system.

In a further embodiment the medical imaging system may further comprise a monitor drive arrangement arranged to control a movement of the monitor ceiling suspension 65 over the loop around the first pair of elongated rails. The monitor drive arrangement may further be arranged to move the monitor ceiling suspension 65 in a direction towards X1 or away X2 from the patient bed which is positioned under the rail system, as schematically shown in Fig. 8 and Fig. 9. The monitor ceiling suspension may be further arranged to rotate an attached monitor around a vertical axis or move the monitor up (increased distance from the floor of the examination room) or down (reduced distance from the floor of the examination room). The medical imaging system may further include a user interface 68 such as a touch screen display to control the movement of the monitor ceiling suspension 65 (see Fig. 11). This allows an interventionalist 115 to position the non-sterile monitor 66 attached to the monitor ceiling suspension to a preferred position, for example to have an optimal viewing angle. During a clinical procedure the required projection of the imaging component (e.g. a C-arc including an X-ray source and a detector or an upper detector robot 70 holding a detector 80) may result in a position that conflicts with the desired position of the monitor. As a consequence, the monitor 66 may have to be moved several times over the loop formed by the rails of the second pair 20 and the curved rail part 30. The control system 111 processing the images obtained with the camera 100 may detect potential conflicts between the present position of the monitor 66 and the desired future position of the imaging component. When the movement of monitor ceiling suspension 65 is motorized, the determined potential conflicts may be automatically resolved by moving the monitor out of the way. This increases the ease-of-use of the medical imaging system in a clinical workflow.

In a further embodiment the control system 111 processing the images obtained with the camera 110 is configured to detect a pose and the location of the interventionalist 115. The control system is further configured to position the monitor 66 in dependence of the detected pose and location of the interventionalist and the location of the imaging component 70, 80.

In a further embodiment the medical imaging system may further comprise a carriage drive arrangement arranged to control a translational movement of the carriage 60 over the first pair of elongated rails 10. The control system 111 is further configured to automate one or more aspects of the operation of the medical imaging system, such as positioning 61 of the carriage 60 using the carriage drive arrangement, positioning of the monitor ceiling suspension (shown in Fig. 11) using the monitor drive arrangement, positioning of the upper detector robot and the lower source robot based on the determined location of the patient 120 or the determined location of the interventionalist or the determined location of the monitor 66 attached to the monitor ceiling suspension to prevent a collision of the upper detector robot or the lower source robot with the patient 120, patient bed 67, interventionalist 115 or monitor 66. The automation realized by the control system 111 enhances the safety of the medical imaging system and relieves the burden on medical staff involved in the clinical procedure.

In a further embodiment the carriage drive arrangement may further include a position sensor for determining the position of the carriage 60. An example of a carriage position sensor is an encoder for encoding the position of the carriage 60 on the first pair of elongated rails 10. The control system 111 is configured to receive the encoder position of the carriage and determine the position of the carriage 60 on the first pair of rails. The monitor drive arrangement may further include a sensor for determining the position of the monitor ceiling suspension. An example of a monitor ceiling suspension position sensor is a position encoder for encoding the position of the monitor ceiling suspension 65 on the second pair of elongated rails. The control system 111 is configured to receive the encoder position of the monitor ceiling suspension and determine the position of the monitor ceiling suspension on the second pair of rails. When the movements of the carriage and the monitor ceiling suspension are motorized and equipped with position sensors, the position as well as the change of position during a movement of the monitor ceiling suspension and carriage is known to the control system. In case the interventionalist steers the imaging component to a desired projection position which is conflicting with the actual position of the monitor, the control system can automatically move the monitor temporarily out of the way and later restore its position when the imaging component is moved away from the conflicting position. This reduces the task of the interventionalist (or staff) to avoid collisions between the imaging component and the monitor.

In case of a medical imaging system comprising an upper detector robot and a lower source robot, the robotic arms may include position encoders that are read out by the control system 111 to determine the actual pose and position of the robot arms. The position of the motorized monitor ceiling suspension can similarly be read out, using encoders. As such, a geometric model of the total setup including the upper detector robot, the lower source robot and monitor coupled to the monitor ceiling suspension may be determined to enable a continuous calculation of a distance between the upper detector robot, the lower source robot and the monitor. The control system is arranged to move the monitor ceiling suspension dependent on the calculated distance to prevent a collision with the robots.

Fig. 11 shows an example of a medical imaging system 100 including an upper detector robot 70 and a lower source robot 71, wherein the upper detector robot and a monitor ceiling suspension 65 are slideably coupled to an integrated rail system. The carriage 60 is slideable over the first pair of rails of which only one rail 10 is shown in Fig. 11. A rail 20 of the second pair of rails is coupled with the side of the rail 10 of the first pair of rails that is facing away from the patient bed 67. The monitor ceiling suspension 65 is slideably coupled to the rail of the second pair. The control system 111 is arranged to move the monitor ceiling suspension to prevent a collision of the monitor 66 with the upper detector robot 70. The control system 111 may be further configured to control the position the radiation detector 80, the radiation source 81 or the monitor 66 in dependence of a user selected position or in dependence of a user selected clinical procedure. A touch screen display 68 attached to the patient bed 67 may be used by the user, for example to select a position on the patient 120 to be imaged, or to select a clinical procedure.

A further aspect of the disclosure provides a method of assembling a rail system for a ceiling mounted medical imaging component. This method comprises the steps of coupling a first pair of elongated rails 10 to a support structure. The rails of the first pair of elongated rails are positioned in parallel and enable a slideably coupling of an imaging component wherein the imaging component may, for example, include a C-arc including an X-ray source and X-ray detector or the imaging component may include an upper detector robot 70 with an attached X-ray detector 80 as shown in Fig. 10 or 11.
The method further comprises coupling a second pair of elongated rails 20 to the support structure. The rails of the second pair are positioned in parallel to the first pair and enable a slideably coupling of a monitor ceiling suspension 65. The rails of the second pair are spatially separated from each other by the first pair of rails. The method further comprises
coupling a curved rail part 30 to the rails of the second pair of elongated rails to create a loop partly or wholly enclosing the first pair of rails and enabling a sliding movement of the monitor ceiling suspension 65 over the loop from one rail of the second pair to the other rail of the second pair;
coupling the curved rail part to the support structure;
coupling the support structure to a ceiling 55;
slideably coupling an imaging component to the first pair of rails;
slideably coupling the monitor ceiling suspension to the second pair of rails.

The order of the steps in the above list may be changed dependent on the actual embodiment. For example, when the support structure is realized with a frame of profiles, the first step of the method may be to couple the support structure to the ceiling to be followed by coupling of the second pair of rails, coupling the curved rail part, coupling of the first pair of rails, slideably coupling of the imaging component to the first pair of rails and slideably coupling of the monitor ceiling suspension to the second pair of rails.

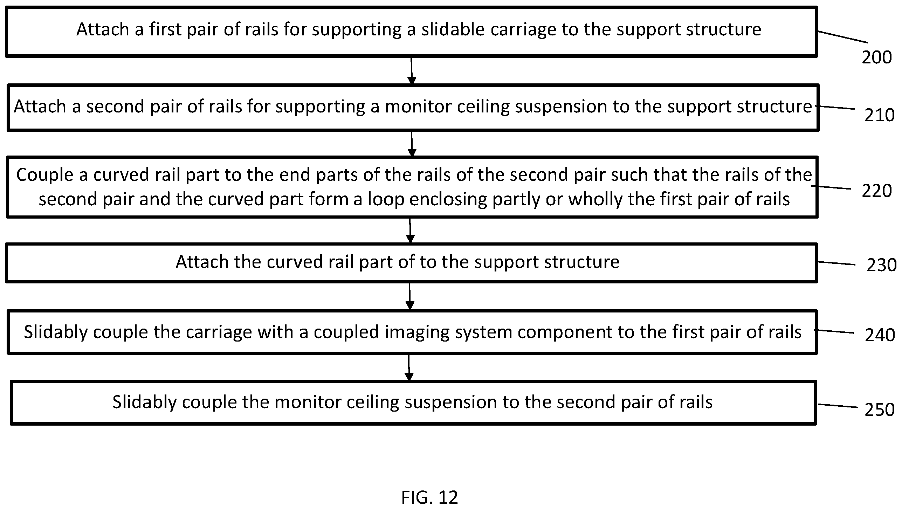

Fig. 12 shows steps of an example of a method of assembling a rail system for a medical imaging system. The method includes a step 200 of attaching a first pair of elongated rails 10 parallel to a support structure. The support structure may include profiles, metal frames, rod interface blocks and rods which are coupled to a ceiling of an examination room. The method further includes a step 210 of attaching a second pair of rails 20 to the support structure. The rails of the second pair are spatially separated, positioned in parallel to the first pair of rails and enclosing the first pair of rails as shown in Fig. 2, Fig. 3 or Fig. 4. The method further includes a step 220 of attaching a curved rail part to couple the ends of the rails of the second pair of rails to create a loop that is partially (shown in Fig. 2) or wholly (shown in Fig. 4) enclosing the first pair of rails 10. In a further step 230 the curved rail part is coupled to the support structure. In next steps 240, 250 a carriage 60 for coupling an imaging component is slideable coupled to the first pair of rails and a monitor ceiling suspension is slideably coupled to a rail of the second pair of rails. Examples of the resulting integrated rail system are shown in Fig. 8 and Fig. 9.

The method of assembling a rail system for a ceiling mounted medical imaging component may further comprise
coupling a further curved rail part 35 to the rails of the second pair 20 of elongated rails, wherein the further curved rail part, the curved rail part and the second pair of elongated rails coupled together form a closed loop around the first pair of rails 10, and
coupling the further curved rail part 35 to the support structure.

The obtained rail system after following the above assembly steps now provides a closed loop of rails to allow the monitor ceiling suspension to be moved around the imaging component which is slideably coupled to the first pair of rails.

The method of assembling a rail system for a ceiling mounted medical imaging component may additionally comprise the coupling of each rail of the second pair 20 of elongated rails with its closest neighboring rail of the first pair 10 of elongated rails. An example of such an obtained rail system 12 is shown in Fig. 7 or Fig. 10.

As illustrated in Fig. 8, 9 or 10 the coupling of a rail of the first pair 10 or a rail of the second pair 20 to the support structure may comprise the steps
coupling the rail to at least one rod interface block 40, and
coupling each of the at least rod interface block with at least one rod 50, 50A, 50B to the ceiling 55.

The present disclosure provides a rail system for a ceiling mounted imaging component comprising a first pair of elongated rails 10 positioned in parallel and configured for slideably coupling an imaging component 60, 70, 80 thereto, as shown for example in Fig. 10. The rail system further includes a second pair of elongated rails 20 positioned in parallel to the first pair and configured for slideably coupling a monitor ceiling suspension 65 thereto. Each rail of the second pair may be coupled back to back with its closes neighboring rail of the first pair of rails as shown in Fig. 8 and 10. The rails of the second pair are spatially separated from each other by the first pair of rails and a curved rail part 30 couples the rails of the second pair to form a loop partly or wholly the imaging component and enabling a monitor 66 attached to the monitor ceiling suspension to be moved over the loop around the patient bed 67 to a position that is not conflicting with a movement 61 of the imaging component and provides a good viewing for a physician 115. The rail system further includes a support structure comprising rods 50, 50A, 50B and rod interface blocks 40 for coupling the first pair of rails, the second pair of rails and the curved rail part to a ceiling 55.

While the invention has been illustrated and described in detail in the drawings and foregoing description, such illustrations and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. Any reference signs in the claims should not be construed as limiting the scope.

## Claims

1. A rail system for a ceiling mounted medical imaging component comprising
a first pair of elongated rails (10) positioned in parallel and configured for slideably coupling an imaging component;
a second pair of elongated rails (20) positioned in parallel to the first pair and configured for slideably coupling a monitor ceiling suspension (65), the rails of the second pair being spatially separated from each other by the first pair of rails;
a curved rail part (30) coupling the rails of the second pair of elongated rails, the curved rail part together with the second pair or elongated rails forming a loop partly or wholly enclosing the first pair of rails; the curved rail part being arranged to allow a sliding movement of the monitor ceiling suspension over the loop;
a support structure coupleable to the ceiling (55), the first pair, second pair and curved rail part being coupled to the support structure.

2. The rail system of claim 1 comprising a further curved rail part (35) coupling the rails of the second pair of elongated rails, wherein the further curved rail part, the curved rail part and the second pair of elongated rails coupled together forming a closed loop around the first pair of rails.

3. The rail system of claim 1 or 2 wherein the support structure comprises a first plurality of rod interface blocks (40) and a second plurality of rods (50, 50A, 50B) arranged for coupling the rod interface block to the ceiling (55), each rod interface block being coupled to the first pair of elongated rails, or to the second pair of elongated rails or to the curved rail part or to the further curved rail part.

4. A medical imaging system (100) comprising the rail system according to anyone of claims 1 to 3, the medical imaging system comprising:
a carriage (60) slideably being coupled to the first pair of elongated rails,
an imaging component of the medical imaging system being coupled to the carriage,
a monitor ceiling suspension (65) being slideably coupled to the second pair of elongated rails,
a monitor (66) being coupled to the monitor ceiling suspension (65)
a patient bed (67) to be positioned on a floor.

5. The medical imaging system of claim 4 wherein the imaging component comprises a C-arc including an X-ray detector and an X-ray source or wherein the imaging component comprises an upper detector robot (70) for movably holding an X-ray detector (80), the medical imaging system further comprising a lower source robot (71) for movably holding an X-ray source (81), the source robot to be positioned on the floor.

6. The medical imaging system according to claim 4 or 5 further comprising a carriage drive arrangement arranged to control a translational movement of the carriage (60) over the first pair of elongated rails and a monitor drive arrangement arranged to control a movement of the monitor ceiling suspension (65) over the loop around the first pair of rails, the loop comprising the second pair of elongated rails and the curved rail part (30).

7. The medical imaging system of claim 6 further comprising a control system (111) configured to control any one or more of the carriage drive arrangement, the monitor drive arrangement, the upper detector robot (70), the lower source robot (71).

8. The medical imaging system according to claim 7 wherein the carriage drive arrangement includes a carriage position sensor arranged to determine the position of the carriage on the first pair of rails and the monitor drive arrangement includes a monitor position sensor arranged to determine the position of the monitor ceiling suspension on the loop, the control system (111) being configured to process data received from the carriage position sensor and the monitor position sensor and control any one or more of the carriage drive arrangement, the monitor drive arrangement, the upper detector robot (70), the lower source robot (71) in dependence of the processed data.

9. The medical imaging system of claim 7 wherein the medical imaging system further comprises
- a camera (110);
- the control system (111) being configured to process the image data obtained by the camera and control any one or more of the carriage drive arrangement, the monitor drive arrangement, the upper detector robot (70) and the lower source robot (71) in dependence of the processed image data.

10. The medical imaging system of claim 8 or 9 wherein the medical imaging system further comprises a touch screen user interface for receiving input on a user selected position of the radiation detector (80), the radiation source (81) or the monitor (66), or receiving input on a user selected clinical procedure, the control system being configured to control the position the radiation detector (80), the radiation source (81) or the monitor (66) in dependence of the received input.

11. The medical imaging system according to claim 9 or 10 wherein the control system (111) is further configured to process the images obtained with the camera and determine a potential position conflict between a present and desired position of anyone or more of an X-ray detector, upper detector robot (70), X-ray source, lower source robot (71), monitor (66), monitor ceiling suspension (65), the control system being further configured to control the positioning of any one or more of the carriage (60), the monitor ceiling suspension (65), the upper detector robot (70) or the lower source robot (71) in dependence of the determined potential position conflict to prevent a collision.

12. A method of assembling a rail system for a ceiling mounted medical imaging component, comprising the steps of
coupling a first pair of elongated rails (10) to a support structure, the rails of the first pair of elongated rails being positioned in parallel and configured for slideably coupling an imaging component;
coupling a second pair of elongated rails (20) to the support structure, the rails of the second pair being positioned in parallel to the first pair and being configured for slideably coupling a monitor ceiling suspension (65), the rails of the second pair being spatially separated from each other by the first pair of rails;
coupling a curved rail part (30) to the rails of the second pair of elongated rails, the curved rail part together with the second pair or elongated rails forming a loop partly or wholly enclosing the first pair of rails; the curved rail part being arranged to allow a sliding movement over the loop;
coupling the curved rail part to the support structure;
coupling the support structure to a ceiling (55);
slideably coupling an imaging component to the first pair of rails;
slideably coupling the monitor ceiling suspension to the second pair of rails.

13. The method of assembling a rail system for a ceiling mounted medical imaging component of claim 12, further comprising
coupling a further curved rail part (35) to the rails of the second pair (20) of elongated rails, wherein the further curved rail part, the curved rail part and the second pair of elongated rails coupled together form a closed loop around the first pair of rails (10),
coupling the further curved rail part (35) to the support structure.

14. The method of assembling a rail system for a ceiling mounted medical imaging component of claim 12 or 13 further comprising
coupling each rail of the second pair (20) of elongated rails with its closest neighboring rail of the first pair (10) of elongated rails.

15. The method of assembling a rail system for a ceiling mounted medical imaging component of claim 12, 13 or 14 wherein the coupling of a rail of the first pair (10) or the second pair (20) to the support structure comprises
coupling the rail to at least one rod interface block (40), and
coupling each of the at least rod interface block with at least one rod (50, 50A, 50B) to the ceiling (55).
